(19) Europäisches Patentamt
European Patent Office
Office européen des brevets

(11) Veröffentlichungsnummer: **0 216 244**
**A2**

# (12) EUROPÄISCHE PATENTANMELDUNG

(21) Anmeldenummer: **86112464.2**

(22) Anmeldetag: **09.09.86**

(51) Int. Cl.[4]: **C07C 155/06**

(30) Priorität: **26.09.85 DE 3534246**

(43) Veröffentlichungstag der Anmeldung: **01.04.87 Patentblatt 87/14**

(84) Benannte Vertragsstaaten: **BE DE FR GB IT NL**

(71) Anmelder: **AKZO N.V.**
**Velperweg 76**
**NL-6824 BM Arnhem(NL)**

(72) Erfinder: **Bergfeld, Manfred, Dr. Dipl.-Chem.**
**August-Pfeffer-Strasse 6**
**D-8765 Erlenbach/Mechenhard(DE)**
Erfinder: **Eisenhuth, Ludwig, Dr. Dipl.-Chem.**
**Lauterhofstrasse 44**
**D-8753 Obernburg(DE)**

(74) Vertreter: **Fett, Günter**
**Akzo GmbH Kasinostrasse 19-23**
**D-5600 Wuppertal 1(DE)**

## (54) Verfahren zur Herstellung von Alkylen-bis-dithiocarbamaten oder deren Ammoniak-Addukten sowie danach herstellbare Gemische.

(57) Die Erfindung betrifft ein Verfahren zur Herstellung von Alkylen-bis-dithiocarbamaten oder deren Ammoniak-Addukten sowie danach herstellbare Gemische. Ausgangsprodukte sind Schwefelkohlenstoff, Ammoniak, ein Diamin und ein Metalloxid, welches kennzeichnungsgemäß Mangan-II-oxid ist, bzw. dessen Hydrat oder Hydroxid. Man läßt das Metalloxid bzw. sein Hydrat oder sein Hydroxid entweder mit einem Gemisch, hergestellt aus einem Alkylendiamin, Schwefelkohlenstoff und Ammoniak in Wasser, oder mit einer wäßrigen Lösung des Ammonium-alkylen-bis-dithiocarbamats reagieren. Die Konzentration der einzelnen Ausgangsstoffe sowohl relativ zueinander als auch bezogen auf Wasser ist entscheidend dafür, welche Verbindungen schließlich im einzelnen erhalten werden. Sie sind hauptsächlich als Fungizide verwendbar.

EP 0 216 244 A2

# Verfahren zur Herstellung von Alkylen-bis-dithiocarbamaten oder deren Ammoniak-Addukten sowie danach herstellbare Gemische

Die Erfindung betrifft ein Verfahren zur Herstellung von Alkylen-bis-dithiocarbamaten oder deren Ammoniak-Addukten ausgehend von Schwefelkohlenstoff, Ammoniak, einem Diamin und einem Metalloxid bzw. dessen Hydrat oder Hydroxid. Die Erfindung betrifft ebenfalls danach herstellbare Gemische. Es handelt sich dabei hauptsächlich um das Mangan-ethylen-bis-dithiocarbamat, auch "Maneb" genannt, und seine Ammoniak-Addukte.

Maneb und auch die Ammoniak enthaltenden Addukte von Maneb sind als Fungizide bekannt. Die Herstellung dieser Verbindungen kann auf diese Weise geschehen, daß man zunächst Ethylendiamin, Schwefelkohlenstoff und eine starke Base, wie z.B. Natronlauge zu Natrium-bis-ethylen-dithiocarbamat umsetzt (US-PS 2,3l7,765). Wäßrige Lösungen derartiger Salze werden dann mit wasserlöslichen Manganverbindungen, wie Mangansulfat oder Manganchlorid umgesetzt (siehe US-PS 2,5O4,4o4), wobei das in Wasser unlösliche Mangan-ethylen-bis-dithiocarbamat ausfällt. Nach diesem Verfahren wird das Maneb gegenwärtig im technischen Maßstab hergestellt.

Aus der DE-PS l2 O2 66 ist weiterhin ein Verfahren zur Herstellung von Ammoniak enthaltenden Addukten des Mangan-Salzes der Ethylen-bis-dithiocarbamitsäure bekannt, bei dem man ein wasserlösliches Salz, z.B. ein Ammonium-Salz der Ethylen-bis-dithiocarbamitsäure mit einem wasserlöslichen Mangansalz zum Beispiel Mangan-Sulfat in Gegenwart von Ammoniak umsetzt. Diese Patentschrift lehrt auch, daß Maneb mit Ammoniak stabile Additionsprodukte bildet. Sie zeigen eine wesentlich bessere Beständigkeit beim Lagern als Maneb. Außerdem haben sie eine gute fungizide Wirksamkeit und eine bessere Verträglichkeit für Pflanzen als das Maneb selbst. Eine Lehre, wie die einzelnen Ammoniak-Addukte mit l oder 2 Molekülen Ammoniak pro Atom Mangan herzustellen sind, wird nicht gegeben.

Aus der Österreichischen Patentschrift l9 54 4O ist schließlich ein Verfahren zur Herstellung von wenig wasserlöslichen, kristallinen Ethylen-bis-dithiocarbamaten mehrwertiger Metalle bekannt, wobei man das Metalloxid entweder mit der nötigen Menge der zur Bildung des Ammonium-dithiocarbamates erforderlichen Ausgangssubstanzen reagieren läßt oder mit einer wäßrigen Lösung von Ammonium-ethylen-bis-dithiocarbamat. An Metalloxiden wird lediglich das Zinkoxid konkret genannt. Auch in den Beispielen wird nur Zinkoxid zur Herstellung von Zink-ethylen-bis-dithiocarbamat eingesetzt. Von Ammoniak-Addukten und deren gezielter Herstellung ist keine Rede.

Aufgabe der Erfindung ist es, ein Verfahren zur Herstellung von Alkylen-bis-dithiocarbamaten und ihren Ammoniak-Addukten zu entwickeln, das es erlaubt, sowohl die ammoniakfreien Verbindungen als auch die ammoniakhaltigen Addukte mit bis zu 2 Mol Ammoniak/l Mol Alkylen-bis-dithiocarbamat gezielt und auf einfache und wirtschaftlich günstige Weise, ausgehend von Schwefelkohlenstoff, Ammoniak, einem Diamin und einer Metallverbindung ohne Isolierung einer Zwischenverbindung herzustellen.

Die erfindungsgemäße Lösung dieser Aufgabe ist den Patentansprüchen zu entnehmen. Die Erfindung ist im wesentlichen dadurch gekennzeichnet, daß man aus der Vielzahl der möglichen Metalloxide ihrer Hydrate bzw. Hydroxide das Manganoxid, sein Hydrat bzw. Hydroxid auswählt und es direkt oder indirekt mit Schwefelkohlenstoff, Ammoniak und einem Alkylendiamin in wäßrigem Medium umsetzt. Darüberhinaus ist es zur Herstellung der jeweiligen Einzelverbindung entscheidend, in welchen Konzentrationen die einzelnen Ausgangsstoffe sowohl relativ zueinander als auch bezogen auf Wasser eingesetzt werden.

Das erfindungsgemäße Verfahren kann entweder in einer einzigen Stufe oder in zwei Stufen durchgeführt werden.

In dem zweistufigen Verfahren wird zunächst aus dem Alkylen-diamin, Schwefelkohlenstoff und Ammoniak das Ammonium-alkylen-bis-dithiocarbamat in wäßriger Lösung hergestellt, wie es zum Beispiel in der FR-PS l O99 969 sowie der US-PS 2 844 623 offenbart wird. In einer zweiten Stufe wird dann diese wäßrige Lösung des Ammonium-alkylen-bis-dithiocarbamats mit Manganoxid in Pulverform umgesetzt, wobei man für eine gute Verteilung des Manganoxids in der wäßrigen Phase sorgt. Dies kann zum Beispiel durch intensives Rühren geschehen. Selbstverständlich kann man auch zuerst eine wäßrige Suspension des Manganoxids herstellen und in diese sodann das Ammoniumsalz zugeben.

Ammonium-alkylen-bis-dithiocarbamat und Mangan-II-oxid können sowohl in stöchiometrischen Verhältnissen umgesetzt werden als auch in nicht-stöchiometrischen, wobei dann ein Überschuß an Ammonium-alkylen-bis-dithiocarbamat bevorzugt wird.

Die Menge an Wasser wird am besten so hoch bemessen, daß man eine gut mischbare Suspension erhält. Es ist zweckmäßig, daß mindestens soviel Wasser bei der Reaktion vorhanden ist, daß eine Ausgangskonzentration des Ammonium-alkylen-bis-dithiocarbamats von höchstens IO Mol/l Wasser gegeben ist.

Die Umsetzung kann durch folgende Reaktionsgleichung wiedergegeben werden:

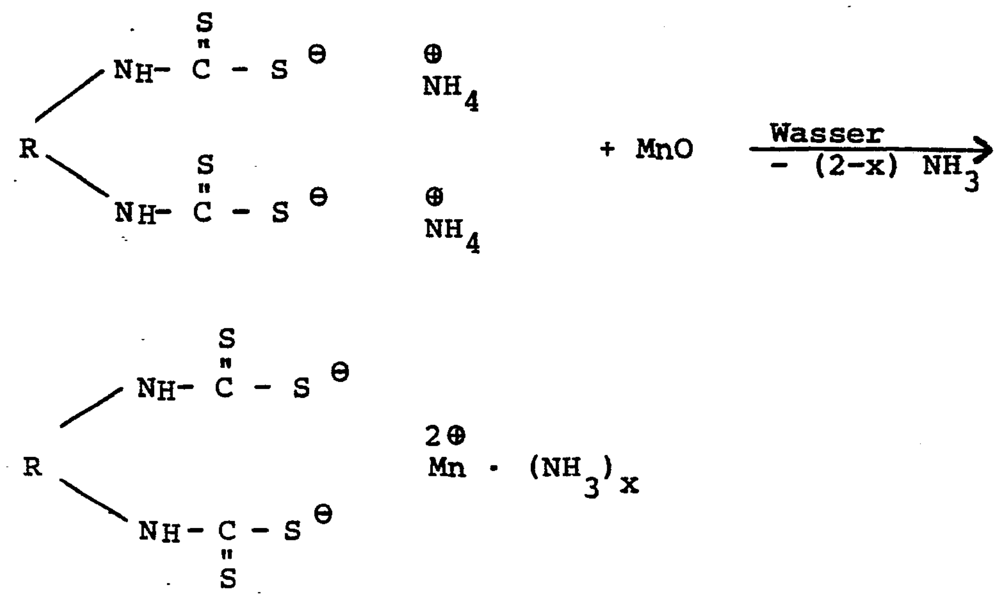


$R = C_2H_4$ bis $C_6H_{12}$

$X = 0$ bis 2

Der Ammoniakgehalt des entstehenden Produktes hängt von der Konzentration des Ammonium-alkylen-bis-dithiocarbamats im Reaktionssystem ab. Je niedriger die Konzentration von Ammonium-alkylen-bis-dithiocarbamats ist, das heißt je höher die Wasserkonzentration im System ist, um so weniger Ammoniak enthält das entstehende Produkt. Bei sehr hohen Wassergehalten kann man ein praktisch ammoniakfreies Produkt erhalten.

Die Zusammenhänge zwischen Konzentration an Ammonium-alkylen-bis-dithiocarbamat und dem Ammoniakgehalt des entstehenden Produktes sind in der folgenden Tabelle am Beispiel von Ammonium-ethylen-bis-dithiocarbamat (AEBDC) Zusammengefaßt:

Tabelle

| AEBDC Mol/l Wasser | Produktanalyse Mol $NH_3$/2 Mol $CS_2$ |
|---|---|
| 0,02 | 0 |
| 0,6 | 1 |
| 1,1 | 1,1 |
| 4,5 | 2 |

Die Reaktion kann zwischen Raumtemperatur und 60°C durchgeführt werden.

Die Reaktionszeit liegt je nach Reaktionstemperatur und Rührintensität bei l-6 h.

Die Reaktionszeit kann dadurch verkürzt werden, daß man möglichst feinteiliges, feinpulveriges Manganoxid verwendet. Auch der Einsatz eines Suspensionshilfsmittels ist vorteilhaft.

Nach beendeter Reaktion wird das Reaktionsgemisch abfiltriert, gegebenenfalls gewaschen und getrocknet.

Es war besonders überraschend, daß man gemäß der Erfindung auf einfache und wirtschaftliche Weise die Mangan-alkylen-bis-dithiocarbamate und deren Ammoniak enthaltende Addukte durch günstige Wahl der Konzentration von Ammonium-alkylen-bis-dithiocarbamate herstellten kann.

Nach dem einstufigen Verfahren wird zunächst zu einer wäßrigen Lösung von Alkylendiamin und Ammoniak unter intensivem Rühren der Schwefelkohlenstoff hinzugefügt, wobei die Temperatur im allgemeinen unter 40°C, insbesondere unter 30°C gehalten wird. Zu einem so aus Diamin, Schwefelkohlenstoff und Ammoniak hergestellten Gemisch gibt man dann pulverförmig oder suspendierend das Manganoxid und rührt weiter, wobei die Reaktionstemperatur im Bereich von 30 bis 60°C liegen kann. Je nach Reaktionstemperatur und Rührintensität liegt die Reaktionszeit bei l-6 h. Das Reaktionsprodukt wird abfiltriert, ggf. mit Wasser gewaschen und getrocknet.

Die Zusammenhänge zwischen den Molverhältnissen an Alkylendiamin, Schwefelkohlenstoff und Ammoniak einerseits und der Adduktbildung andererseits sind in der folgenden Tabelle am Beispiel von Ethylendiamin zusammengefaßt.

Tabelle

| Ethylendiamin/$CS_2$/$NH_3$ Mol / Mol / Mol | | | Mol $NH_3$/l$H_2O$ | Produktanalyse Mol $NH_3$/2 Mol $CS_2$ |
|---|---|---|---|---|
| 1 | 2 | 1 | 0,15 | 0 |
| 1 | 2 | 1 | 0,6 | 0 |
| 1 | 2 | 1 | 10 | 0,9 |
| 1 | 2 | 1,2 | 12 | 1 |
| 1 | 2 | 4 | 19 | 1.84 |

Es war besonders überraschend, daß man gemäß der Erfindung auf einfache und wirtschaftliche Weise die Ammoniak-Addukte der Mangan-Salze durch günstige Wahl der Ammoniak-Konzentration herstellen kann, insbesondere daß nicht die zur Bildung des Ammonium-dithiocarbamats erforderlichen molaren Mengen notwendig sind.

Die erhaltenen Produkte werden durch IR-Messung, Elementar-Analyse, $CS_2$-Bestimmung nach der Methode aus CIPAC Handbook, 1970, I, 463 und $NH_3$-Bestimmung (titrimetrische Bestimmung des mit Natronlauge freizusetzenden Ammoniaks) charakterisiert.

Bei einem Einsatz von 80%igem Manganoxid wurden Produktausbeuten bis nahe 100% erzielt; der Gehalt an ammoniakhaltigem Maneb betrug bis zu 85%. Das entstehende Produkt-Gemisch ist direkt einsetzbar.

In beiden Verfahrensweisen kann als Manganoxid ein Manganoxid technischer Qualität verwendet werden, das vorzugsweise ein Manganoxidgehalt von 70 bis 90 Gew.-% aufweist. Insbesondere wird ein Manganoxid technischer Qualität mit folgender Zusammensetzung eingesetzt: MnO 78-80%, $MnO_2$ ca. 0,5%, $Al_2O_3$ ca. 6%, FeO ca. 5%, $SiO_2$ ca. 3%, C ca. 1%, $K_2O$ ca. 0,6%; der Rest besteht aus einer Vielzahl von Bestandteilen in einer Größenordnung von weniger als 0,5%. Es sind zwar auch die unlöslichen Begleitstoffe des Manganoxids im Endprodukt enthalten, was aber kaum stört, da das Mangansalz ohnehin im allgemeinen - mit Füllstoffen versehen -im Handel ist. Falls die Begleitstoffe jedoch stören, kann man erfindungsgemäß auch reines Manganoxid verwenden. In beiden erfindungsgemäßen Verfahren wird das Manganoxid in möglichst feinkörniger Form verwendet. Besonders vorteilhaft ist es, mit einer Körnung von unter 125 µm zu arbeiten. Derartige Manganoxidproben können gegebenenfalls durch Mahlen und Sieben aus den üblichen Manganoxidmustern erhalten werden.

Es ist vorteilhaft, wenn die Umsetzung unter intensivem Rühren in einem wäßrigen Medium durchgeführt wird. Vorteilhaft ist auch die Gegenwart grenzflächenaktiver Mittel.

Als Diamin können aliphatische Diamine mit 2 bis 6 C-Atomen eingesetzt werden.

Vorzugsweise wird ein aliphatisches 1,2-Diamin, insbesondere Ethylendiamin oder Propylen-diamin verwendet.

Beide Verfahrensvarianten haben den Vorteil, daß die Mutterlauge im Kreislauf geführt werden kann, was insbesondere eine beachtliche Materialeinsparung mit sich bringt. Außerdem ist es möglich, technische Qualitäten von Manganoxid zu verwenden. Das erhaltene Produkt kann direkt als Fungizid verwendet werden. Es ist auch möglich, die Produkte mit anderen Verbindungen zu verschneiden, wie zum Beispiel Zink-ethylen-bis-dithiocarbamat. Das erhaltene Produkt kann auch mit Formaldehyd umgesetzt werden.

Die Erfindung wird durch folgende Beispiele näher erläutert:

<u>Beispiel 1</u>

Herstellung eines Ammoniak-Addukts von Mangan-ethylen-bis-(dithiocarbamat).

In einem Glasreaktionsgefäß wird zu einer Lösung aus 1,12 Mol Ethylendiamin und 1,12 Mol Ammoniak in 232 g Wasser sowie wenigen Tropfen Serdox NOP 9 (Nonylphenylpolyethylenglykol (Chemische Fabrik Servo b.v. Delden/NL)), 2,24 Mol Schwefelkohlenstoff unter intensivem Rühren addiert, wobei die Temperatur unter 30°C gehalten wird. Nach 15 min. fortgesetztem Rühren wird 91 g technisches Manganoxid (MNO-Gehalt: ca. 80%) hinzugefügt und 2,5 h intensiv gerührt, wobei die Temperatur von 40°C mit fortschreitender Reaktion auf 50°C erhöht wird.

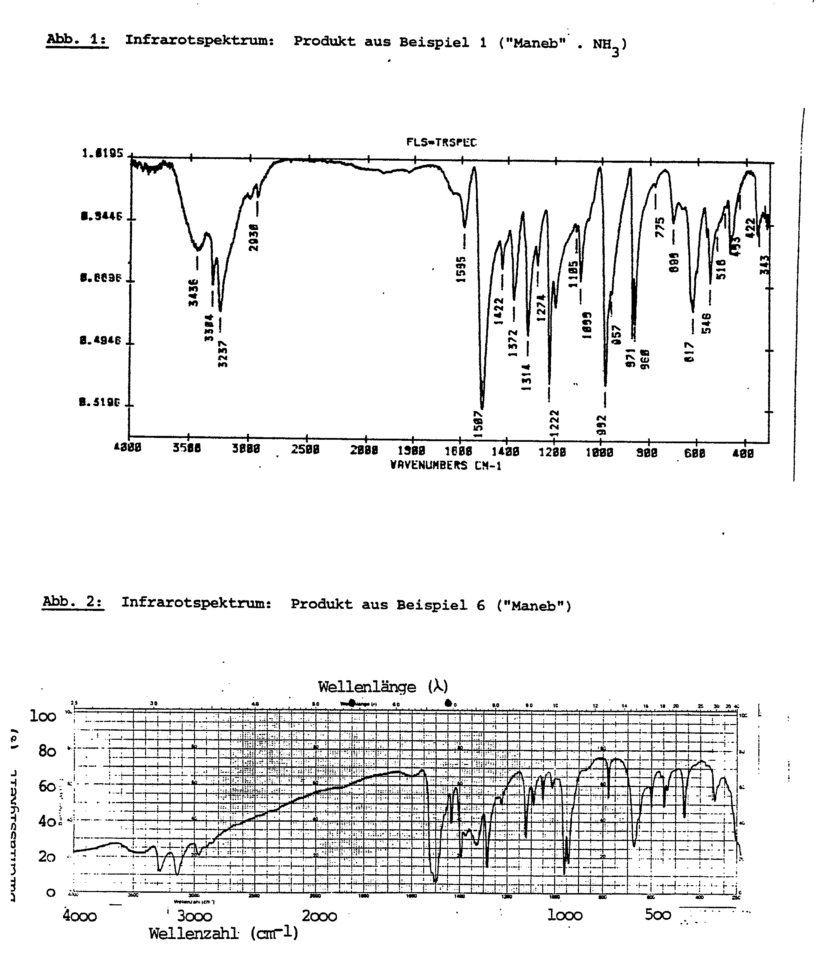

Der Niederschlag wird abfiltriert, mit Wasser gewaschen und bei 50°C getrocknet. Man erhält auf diese Weise 272 g eines Produktes, das in seiner Infrarotanalyse (Abb. 1) identisch ist mit dem Ammoniak-Addukt des Mangan-ethylen-bis-(dithiocarbamat) (Molverhältnis 1 : 1), das im DP 1 202 266 beschrieben ist. Die Produktreinheit liegt bei 85,5% (bestimmt über $CS_2$-Analyse).

| | | |
|---|---|---|
| <u>Analyse:</u> | C | 16,3% |
| | H | 3,1% |
| | N | 13,3% |
| | $CS_2$ | 46,1 |
| | $NH_3$ | 4,1 |

+ Nonylphenylpolyethylenglykol (Chemische Fabrik Servo b.v. Delden/NL)

Daneben enthält das Produkt kleine Mengen der im eingesetzten technischen Manganoxid enthaltenen Begleitstoffe, insbesondere die Oxide von Aluminium, Eisen und Silizium.

Die Mutterlauge enthält noch kleine Mengen der nicht umgesetzten Ausgangsstoffe Schwefelkohlenstoff, Ethylendiamin und Ammoniak und kann daher erneut eingesetzt werden.

Beispiel 2

Herstellung eines Ammoniak-Addukts von Mangan-ethylen-bis-(dithiocarbamat).

Zu einer Lösung aus 1,12 Mol Ethylendiamin und 1,12 Mol Ammoniak in 232 g Wasser sowie wenigen Tropfen Serdox NOP 9 werden unter intensivem Rühren 2,24 Mol Schwefelkohlenstoff zugegeben. Anschließend werden 101 g technisches Manganoxid (MnO-Gehalt: ca. 80%) zugesetzt und das Reaktionsgemisch 4 h bei 40°C intensiv durchmischt. Der Niederschlag wird abfiltriert, gewaschen und getrocknet. Ausbeute: 313 g. Laut Infrarotanalyse liegt wieder das Ammoniak-Addukt des Mangan-ethylen-bis-(dithiocarbamats) vor - (Reinheit 84,2% laut $CS_2$-Analyse).

Analyse:

| | |
|---|---|
| C | 16,2% |
| H | 3,0% |
| N | 13,0% |
| $CS_2$ | 45,4% |
| $NH_3$ | 4,3% |

Beispiel 3

Herstellung eines Ammoniak-Addukts von Mangan-ethylen-bis-(dithiocarbamat).

Eine nach dem in der US-PS 28 44 623 in Spalte 3, ab Zeile 4 beschriebenen Beispiel aus 0,28 Mol Ethylendiamin, 0,56 Mol Schwefelkohlenstoff, 0,56 Mol Ammoniak und 470 g Wasser sowie wenige Tropfen Serdox NOP 9 hergestellte wäßrige Lösung von Ammonium-ethylen-bis-(dithiocarbamat) wird mit 17,1 g technischem Manganoxid (ca. 80%ig, MnO-Gehalt ca. 0,19 Mol) unter Rühren zur Reaktion gebracht. Die Reaktionstemperatur beträgt 50°C, die Reaktionszeit 3 h. Der gebildete Feststoff wird abfiltriert, gewaschen und getrocknet. Man erhält 42,4 g eines Produktes, das laut Infrarotanalyse dem Addukt aus Mangan-ethylen-bis-dithiocarbamat) mit Ammoniak - (Molverhältnis 1 : 1) entspricht (Gehalt: 72,5%).

Analayse:

| | |
|---|---|
| C | 14,6% |
| H | 2,7% |
| N | 11,0% |
| $CS_2$ | 39,0% |
| $NH_3$ | 3,9% |

Beispiel 4

Herstellung eines Ammoniak-Addukts von Mangan-ethylen-bis-(dithiocarbamat).

Zu einer Lösung bestehend aus 0,56 Mol Ethylendiamin, 2,24 Mol Ammoniak und 125 g Wasser werden unter intensivem Rühren 1,12 Mol Schwefelkohlenstoff addiert, wobei die Reaktionstemperatur unter 30°C gehalten wird. Nach 10 min. fortgesetztem Rühren werden 50,5 g techni-

sches MnO (MnO-Gehalt ca. 0,56 Mol) hinzugefügt und das Gemisch 2,5 h bei 50°C weitergerührt. Anschließend wird der Feststoff abfiltriert, gewaschen und getrocknet, wobei 156,1 g eines Produktes erhalten werden, das aufgrund seiner analytischen Daten einem Addukt aus Mangan-ethylen-bis-(dithiocarbamat) mit Ammoniak (Molverhältnis: 1:2) entspricht (Gehalt: 81,1% laut $CS_2$-Analyse).

| Analyse: | | |
|---|---|---|
| | $CS_2$ | 41,3% |
| | $NH_3$ | 8,5% |

Beispiel 5

Herstellung eines Ammoniak-Addukts von Mangan-ethylen-bis-(dithiocarbamat).

Eine nach dem in der US-PS 28 44 623 in Spalte 3 ab Zeile 4 beschriebenen Beispiel aus 0,56 Mol Ethylendiamin, 1,12 Mol Schwefelkohlenstoff, 1,12 Mol Ammoniak und 125 g Wasser hergestellte wäßrige Lösung von Ammonium-ethylen-bis-(dithiocarbamat) wird mit 25 g technischem Manganoxid (80%ig, MnO-Gehalt ca. 0,28 Mol) unter kräftigem Rühren zur Reaktion gebracht. Die Reaktionstemperatur beträgt 50°C, die Reaktionszeit 3 h. Das gebildete Produkt wird abfiltriert, mit Wasser gewaschen und getrocknet. Man erhält 81,7 g eines Produktes, das aufgrund seiner analytischen Daten einem Addukt aus Mangan-ethylen-bis-(dithiocarbamat) mit Ammoniak (Molverhältnis 1 : 2) entspricht (Gehalt: 80,2% laut $CS_2$-Analyse).

| Analyse: | | |
|---|---|---|
| | $CS_2$ | 40,9% |
| | $NH_3$ | 8,6% |

Beispiel 6

Herstellung von Mangan-ethylen-bis-(dithiocarbamat).

Eine nach dem in der US-PS 28 44 623 in Spalte 3 ab Zeile 4 beschriebenen Beispiel aus 0,11 Mol Ethylendiamin, 0,22 Mol Schwefelkohlenstoff, 0,22 Mol Ammoniak und 720 g Wasser hergestellte wäßrige Lösung von Ammonium-ethylen-(bisdithiocarbamat) wird mit 5,2 g technischem Manganoxid (ca. 80%ig, MnO-Gehalt ca. 0,056 Mol) umgesetzt. Die Reaktionstemperatur beträgt 50°C, die Reaktionszeit 3 h. Der Feststoff wird abfiltriert, gewaschen und getrocknet. So wurden 13,7 g eines Produktes erhalten, das in seinem Infrarotspektrum identisch ist mit Mangan-ethylen-bis-(dithiocarbamat) (Abb. 2). Die Produktreinheit liegt laut $CS_2$-Analyse bei 69,8% (Ammoniakanteil: < 0,1%)

Beispiel 7

Herstellung von Mangan-ethylen-bis-(dithiocarbamat.

Zu einer Lösung bestehend aus 0,22 Mol Ethylendiamin, 0,22 Mol Ammoniak und 360 g Wasser sowie wenigen Tropfen Serdox NOP 9 werden unter intensivem Rühren 0,44 Mol Schwefelkohlenstoff addiert. Nach kurzem Rühren werden 10,5 g technisches Manganoxid (80%ig, MnO-Gehalt ca. 0,116 Mol) zugegeben und 6 h weitergerührt, wobei die Temperatur mit fortschreitender Reaktion von 40°C auf 50°C erhöht wird. Anschließend wird der Feststoff abfiltriert, gewaschen und getrocknet. So werden 27,4 g eines Produktes erhalten, das in seinem Infrarotspektrum ammoniakfreiem Mangan-ethylen-bis-(dithiocarbamat) entspricht (Gehalt nach $CS_2$-Analyse 71,4%; Ammoniak-Gehalt < 0,1%).

Beispiel 8:

Herstellung eines Ammoniakaddukts von Manganpropylen-bis-dithiocarbamat.

Zu einer Lösung aus 0,56 Mol Propylendiamin und 0,56 Mol Ammoniak in 116 g Wasser werden unter intensivem Rühren 1,12 Mol Schwefelkohlenstoff addiert, wobei die Temperatur unter 30°C gehalten wird. Nach 15 min. fortgesetztem Rühren

werden 5O,5 g technisches Manganoxid (ca. 8O%ig, MnO-Gehalt ca. 0,56 Mol) addiert und 4 h bei 4O°C intensiv weitergerührt. Der gebildete Feststoff wird abfiltriert, gewaschen und getrocknet.

Analyse: $CS_2$: 35,1%
$NH_3$: 2,2%

## Ansprüche

I. Verfahren zur Herstellung von Alkylen-bis-dithiocarbamten oder deren Ammoniak-Addukten ausgehend von Schwefelkohlenstoff, Ammoniak, einem Diamin und einem Metalloxid, bzw. dessen Hydrat oder Hydroxid, wobei man das Metalloxid bzw. sein Hydrat oder sein Hydroxid entweder mit einem Gemisch, hergestellt aus einem Alkylendiamin, Schwefelkohlenstoff und Ammoniak in Wasser, oder mit einer wäßrigen Lösung des Ammonium-alkylen-bis-dithiocarbamats reagieren läßt, dadurch gekennzeichnet, daß als Metalloxid, bzw. sein Hydrat oder Hydroxid das Mangan-II-oxid bzw. sein Hydrat oder sein Hydroxid verwendet wird.

2. Verfahren nach Anspruch I, dadurch gekennzeichnet, daß man zur Herstellung von ammoniakfreiem Mangan-alkylen-bis-dithiocarbamat ein wäßriges Gemisch aus Alkylendiamin, Schwefelkohlenstoff und Ammoniak im Molverhältnis I/2/O.5-I einsetzt und die Wassermenge so bemißt, daß die Ammoniakkonzentration geringer als O.7 Mol/l Wasser ist.

3. Verfahren nach Anspruch I, dadurch gekennzeichnet, daß man zur Herstellung von ammoniakfreiem Mangan-alkylen-bis-dithiocarbamat das Ammonium-Alkylen-bis-dithiocarbamat in einer Konzentration von weniger als O,2 Mol/l Wasser einsetzt.

4. Verfahren nach Anspruch I, dadurch gekennzeichnet, daß man zur Herstellung des Adduktes von Ammoniak und Mangan-alkylen-bis-dithiocarbamat im Molverhältnis O.8-I,2/I ein wäßriges Gemisch aus Alkylendiamin, Schwefelkohlenstoff und Ammoniak im Molverhältnis I/2/O,5-I einsetzt und die Wassermenge so bemißt, daß die Ammoniakkonzentration größer als I Mol/l Wasser ist.

5. Verfahren nach Anspruch I, dadurch gekennzeichnet, daß man zur Herstellung des Adduktes von Ammoniak und Mangan-alkylen-bis-dithiocarbamat im Molverhältnis O.8-I,2/I das Ammonium-alkylen-bis-dithiocarbamat in einer Konzentration von O,5 bis 2 Mol/l Wasser einsetzt.

6. Verfahren nach Anspruch I, dadurch gekennzeichnet, daß man zur Herstellung des Adduktes von Ammoniak und Mangan-alkylen-bis-dithiocarbamat im Molverhältnis von I.6-2/I das Ammonium-alkylen-bis-dithiocarbamat in einer Konzentration von mehr als 4 Mol/l Wasser einsetzt.

7. Verfahren nach Anspruch I, dadurch gekennzeichnet, daß man zur Herstellung des Adduktes von Ammoniak und Mangan-alkylen-bis-dithiocarbamat im Molverhältnis I.6-2/I die Ausgangsprodukte Alkylendiamin, Schwefelkohlenstoff und Ammoniak im Molverhältnis I/2/ > 2 einsetzt und die Wassermenge so bemißt, daß die Ammoniakkonzentration größer ist als I Mol/l Wasser.

8. Verfahren nach einem oder mehreren der vorangehenden Ansprüche, dadurch gekennzeichnet, daß das Alkylendiamin ein aliphatisches I,2-Diamin mit 2 bis 6 C-Atomen mit insbesondere primären Aminogruppen ist.

9. Verfahren nach einem oder mehreren der vorangehenden Ansprüche, dadurch gekennzeichnet, daß das Alkylendiamin Ethylendiamin ist.

IO. Verfahren nach einem oder mehreren der vorangehenden Ansprüche, dadurch gekennzeichnet, daß das Alkylendiamin Propylen-diamin ist.

II. Verfahren nach einem oder mehreren der vorangehenden Ansprüche, dadurch gekennzeichnet, daß das Manganoxid von technischer Qualität mit einem MnO-Gehalt von 7O bis 9O% ist.

I2. Mangan-alkylen-bis-dithiocarbamat-Gemisch, dadurch gekennzeichnet, daß es nach Anspruch II herstellbar. ist.

**Abb. 1:** Infrarotspektrum: Produkt aus Beispiel 1 ("Maneb" . $NH_3$)

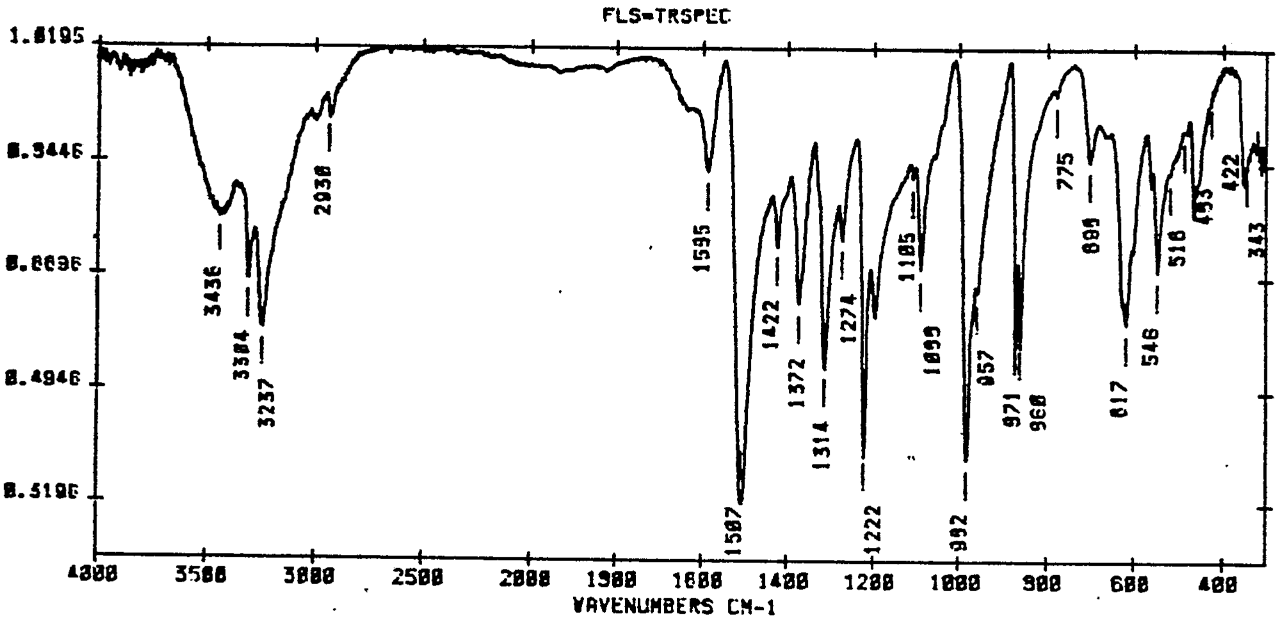


**Abb. 2:** Infrarotspektrum: Produkt aus Beispiel 6 ("Maneb")